# EUROPEAN PATENT SPECIFICATION

(11) **EP 2 728 010 B1**
(45) Date of publication and mention of the grant of the patent: **16.11.2016**
(21) Application number: 11869069.2
(22) Date of filing: 01.07.2011
(51) Int. Cl.: C12P 19/34, C12Q 1/68, C12M 1/38, C07H 21/00, B01L 7/00

(54) **THERMAL CONVECTION POLYMERASE CHAIN REACTION DEVICE**
POLYMERASEKETTENREAKTIONSVORRICHTUNG MIT THERMISCHER KONVEKTION
DISPOSITIF DE RÉACTION EN CHAÎNE DE LA POLYMÉRASE À CONVECTION THERMIQUE

(43) Date of publication of application: 07.05.2014
(73) Proprietor: GeneReach Biotechnology Corp., Taichung City 407, Taiwan (TW)
(72) Inventor: SU, Cheng, Taichung Taiwan (TW); TENG, Ping Hua, Taichung Taiwan (TW)
(74) Representative: Viering, Jentschura & Partner mbB Patent- und Rechtsanwälte
(86) International application number: PCT/CN2011/001093
(87) International publication number: WO 2013/003976

(56) References cited:
- EP-B1- 1 434 880
- WO-A1-2007/028860
- CN-A- 101 003 825
- CN-A- 101 328 503
- US-B2- 7 238 517
- WEN CHOU ET AL: "Rapid DNA amplification in a capillary tube by natural convection with a single isothermal heater", BIOTECHNIQUES, vol. 50, no. 1, 1 January 2011 (2011-01-01), pages 52-57, XP055170196, ISSN: 0736-6205, DOI: 10.2144/000113589

## Description

### BACKGROUND OF THE INVENTION

### 1. Field of the Invention

The present invention relates generally to a polymerase chain reaction device, and more particularly to a thermal convection polymerase chain reaction device.

### 2. Description of the Related Art

In the field of biotechnology, polymerase chain reaction (PCR) is a well-known technology used to amplify specific nucleic acid sequences. The PCR process comprises three major steps including denaturation, primer annealing and extension, which require different reaction temperatures. The required temperature for the denaturation step is typically in a range between 90° C. and 97°. C. The required temperature for the primer annealing step depends on the melting temperature of the primer used. Typically, the annealing temperature ranges from 35° C. to 65° C. The required temperature for the extension step is typically about 72° C.

Convection PCR is generally performed by immersing the bottom of a test tube which contains a PCR mixed solution into a hot water in such a way that the rest portion of the test tube is exposed to atmosphere at room temperature for heat dissipation. As a result, the temperature of the PCR mixed solution will gradually decrease from the bottom of the PCR mixed solution having a temperature of about 97° C. toward the liquid level of the PCR mixed solution having a temperature of about 35° C. Because of the temperature gradient, the heat convection is induced; such that the PCR mixed solution will flow through various regions having different temperatures and then undergo different reaction steps.

In the conventional convective PCR device, high-temperature vapor generated above the surface of the hot water will convectively flow upwardly and then affect the heat dissipation around the middle and upper sections of the test tube, resulting in that the temperature at the level of the PCR mixed solution may not be lowered enough to the required temperature for conducting the primer annealing step. In addition, fluorescence is commonly used to detect the completion of PCR reaction. That is, a fluorescent dye is added into the PCR mixed solution and a laser ray is used to stream through the bottom of the test tube to the PCR mixed solution to detect the intensity of the fluorescence light. In the conventional device for convective PCR, since the bottom of the test tube is immersed in the hot water for being heated, the hot water will badly affect the laser ray, making fluorescent detection impossible.

### SUMMARY OF THE INVENTION

The present invention has been accomplished in view of the above-noted circumstances. It is the primary objective of the present invention to provide a thermal convection polymerase chain reaction device, which can minimize the influence, which is caused by hot air generated from the heat source, on the heat dissipation of middle and upper sections of the test tube.

Another objective of the present invention is to provide a thermal convection polymerase chain reaction device, which is suitable for fluorescent detection of PCR reaction.

To achieve the above-mentioned objectives, the device provided by the present invention is adapted for holding a test tube in which insulated isothermal polymerase chain reaction is performed, which comprises an adiabatic seat and a heating seat. The adiabatic seat has a body provided with a chamber for receiving a bottom of the test tube, a side channel communicated between the chamber and an ambient environment, and an upper channel communicated between the chamber and the ambient environment for insertion of the test tube. The heating seat is inserted into the side channel for stopping at the bottom of the test tube and introducing heat energy into the bottom of the test tube. The heating seat is an electrical heater which contacts and heats the bottom of the test tube, and a gap between the upper channel and the test tube is small. By this way, the influence caused by hot air generated from the heat source on the heat dissipation of the middle and upper sections of the test tube can be reduced and the device of the present invention is suitable for fluorescent detection of PCR reaction.

### BRIEF DESCRIPTION OF THE DRAWINGS

The present invention will become more fully understood from the detailed description given herein below and the accompanying drawings which are given by way of illustration only, and thus are not limitative of the present invention, and wherein:
FIG. 1 is a perspective view of a thermal convection polymerase chain reaction device according to a preferred embodiment of the present invention;
FIG. 2 is another perspective view of the thermal convection polymerase chain reaction device according to the preferred embodiment of the present invention;
FIG. 3 is a cross-sectional view taken along line 3-3 of FIG. 1; and
FIG. 4 is a schematic view showing the movement of the heating seat.

**Reference number**

| | | |
|---|---|---|
| 10 thermal convection polymerase chain reaction device | | |
| 12 test tube | 121 bottom | 122 middle section |
| | 123 upper section | |
| 20 adiabatic seat | 22 body | 24 chamber |
| | 261 side channel | 262 upper channel |
| | 263 lower channel | |
| 30 heating seat | | |
| 40 light unit | 43 filter | |
| 50 dissipating seat | 52 main body | 54 through hole |
| | 541 relatively big diameter section | |
| | 542 relatively small diameter section | |
| | 56 reaction chamber | |
| 60 tube rack | 62 receiving hole | |
| 70 drive | | |
| 80 optical fiber | | |
| 90 photo-sensing device | | |
| P1 contact position | | |
| P2 release position | | |

### DETAILED DESCRIPTION OF THE INVENTION

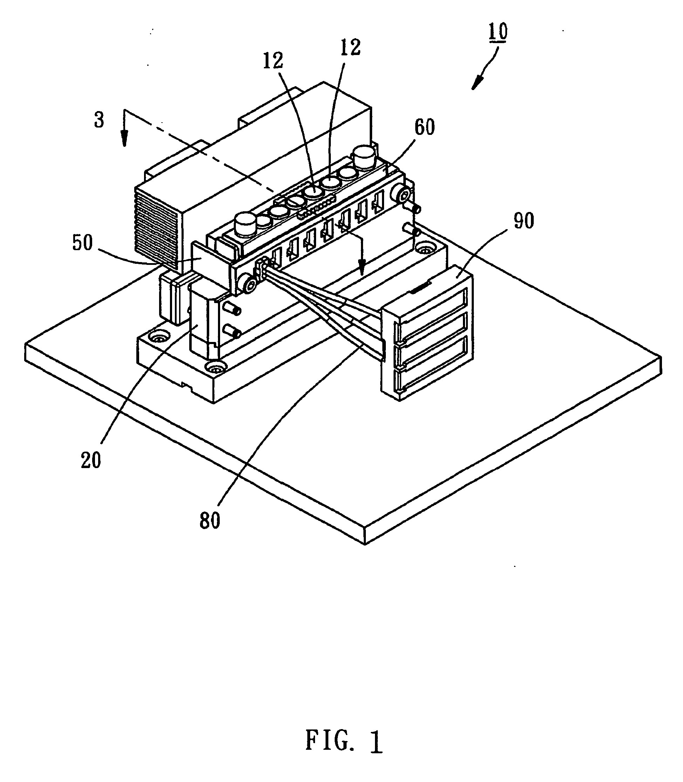

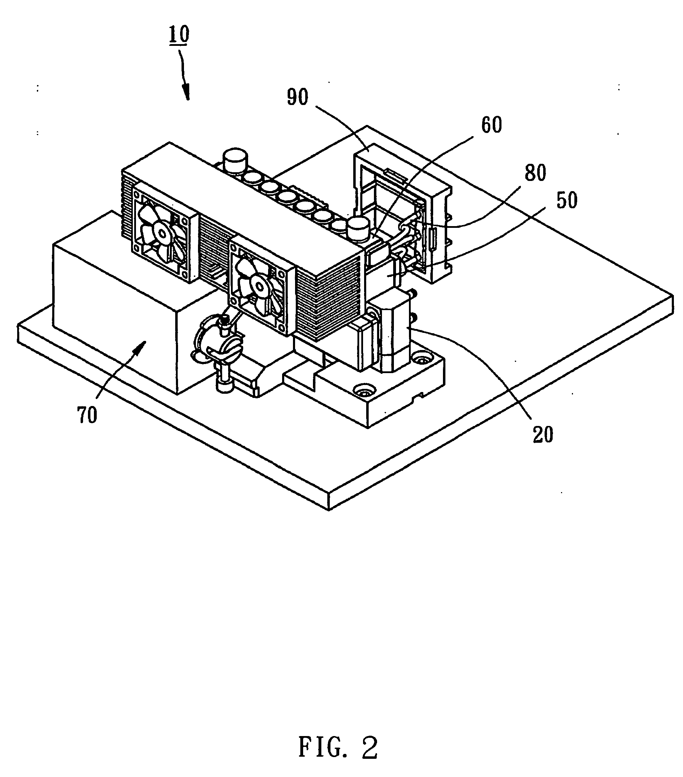

As shown in FIGS. 1-3, a thermal convection polymerase chain reaction device 10 for insulated isothermal PCR, which is provided according to a preferred embodiment of the present invention, mainly comprises an adiabatic seat 20, a heating seat 30, a light unit 40, a dissipating seat 50, a tube rack 60 and a drive 70.

The insulating mount 20 includes a body 22 provided at an inside thereof with a chamber 24 for receiving a bottom 121 of a test tube 12, a side channel 261 communicated with the chamber 24 to an ambient environment, and an upper channel 262 communicated with the chamber 24 to the ambient environment for insertion of the test tube 12. The heating seat 30 has an end portion inserted into the side channel 261 of the adiabatic seat 20 for stopping at the bottom 121 of the test tube 12.

The adiabatic seat 20 is made from a plastic material or a ceramic material. For the plastic material, nylon-glass fiber composite or acrylic-ABS (Acrylonitrile Butadiene Styrene) composite having a low thermal conductivity can be used. The heating seat 30 is made of metal, such as copper; therefore, the heating seat 30 has a high thermal conductivity. The heating seat 30 adopts electricity to generate heat energy, thereby preventing the generation of the high-temperature vapor. In addition, the gap between the upper channel 262 and the test tube 12 is small; therefore, even if the heating seat 30 heats the air within the chamber 24, the heated air will have minor influence on the heat dissipation of the middle and upper sections 122 and 123 of the test tube 12.

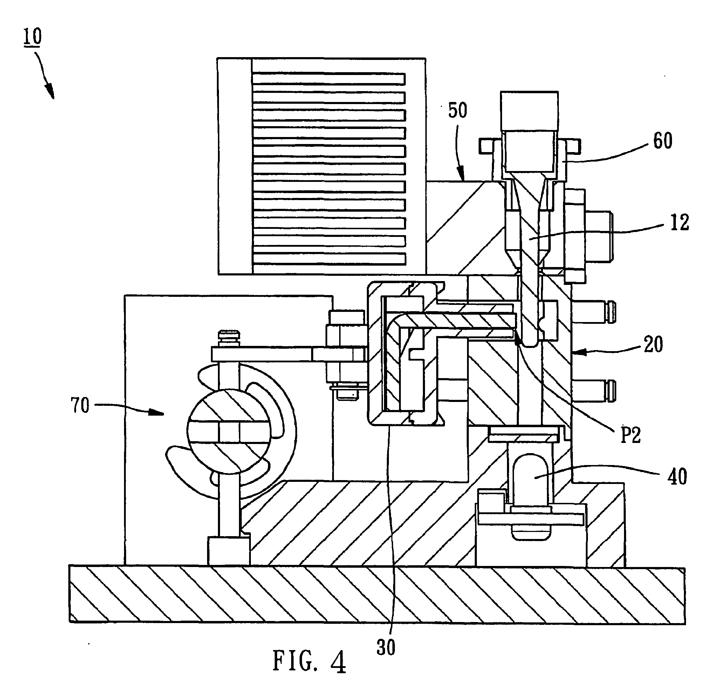

In order to detect the resultant of reaction in every cycling of the PCR, i.e. in order to perform the so-called fluorescent detection of PCR, the light unit 40 provided by the preferred embodiment of the present invention is arranged below the adiabatic seat 20, as shown in FIG 3. In addition, the adiabatic seat 20 is further provided with a lower channel 263 for enabling entrance of the light emitted from the light unit 40 into the chamber 24. The light having a specific wavelength and emitted by the light unit 40 will stream through the lower channel 263 to the PCR mixed solution contained in the test tube 12 to induce the particles having fluorescence characteristic in the PCR mixed solution to emit fluorescent light. By means of using an optical fiber 80 and a photo-sensing device 90 to detect the intensity of the fluorescent light in the test tube 12, the resultant of reaction in the PCR mixed solution can be quantified.

Instead of using hot water, the present invention adopts the heating seat 30 to heat the bottom 121 of the test tube 12; therefore, the light emitted from the light unit 40 will not be affected by hot water. In addition, because the heating seat 30 is arranged at a lateral side of the test tube 12, the light unit 40 is able to be arranged below the test tube 12; therefore, the light emitted from the light unit 40 can stream on the whole test tube. In light of this, the thermal convection polymerase chain reaction device 10 provided by the present invention is suitable for the fluorescent detection of PCR, thereby achieving the objectives of the present invention.

In practice, the light unit 40 can be realized by an LED module, a halogen lamp, a tritium lighting unit or a xenon arc lamp. In addition, a filter 43 can be arranged between the chamber 24 and the light unit 40 to filter the light emitted from the light unit 40 for allowing the light having a specific wavelength to pass therethrough and stream on the test tube 12.

In order to enhance the heat dissipating effect at the middle and upper sections 122 and 123 of the test tube 12, the dissipating seat 50 is further provided in the preferred embodiment of the present invention. As shown in FIG 3, the dissipating seat 50 includes a main body 52 provided with a through hole 54 penetrating therethrough. The main body 52 is mounted on the adiabatic seat 20 in such a way that the through hole 54 is in alignment with the upper channel 262 of the adiabatic seat 20 for insertion of the test tube 12. In PCR process, because the middle and upper sections 122 and 123 are located inside the through hole 54 of the dissipating seat 50 and the dissipating seat 50 is made of a metal material having a high heat transfer coefficient, such as aluminum alloy or copper alloy, the heat energy of the PCR mixed solution in the test tube 12 will be transferred through the air surrounding the test tube 12 to the dissipating seat 50 for further heat dissipation. As a result, when the PCR mixed solution convectively flows upwardly, the PCR mixed solution will gradually cool. Specifically speaking, when the PCR mixed solution flows to the middle section 122 of the test tube 12, the PCR mixed solution can be cooled to a temperature of about 72°C, which is the required temperature suitable for conducting the extension step. When the PCR mixed solution flows to the liquid level, the PCR mixed solution can be further cooled to a temperature of about 35° C., which is lower than the required temperature for conducting the primer annealing step. By this repeated cycling of convection flow, the polymerase chain reaction will continuously run.

In fact, the chamber 24 of the adiabatic seat 20, the upper channel 262 of the adiabatic seat 20 and the through hole 54 of the dissipating seat 50 combinedly form a reaction chamber 56 and the heat energy in the reaction chamber 56 will be transferred to the ambient environment through the dissipating seat 50. In general, the heating seat 30 introduces heat energy into the bottom 121 of the test tube 12, and the dissipating seat 50 transmits the heat energy at the middle and upper sections 122 and 123 of the test tube 12 and the heat energy from the hot air in the upper channel 262 of the adiabatic seat 20 to the ambient atmosphere, such that the PCR mixed solution in the test tube 20 that is held in the reaction chamber 56 and the ambient air surrounding the test tube 12 will have a temperature gradually and upwardly decreasing.

In other words, the adiabatic seat 20 prohibits heat exchange between the reaction chamber 56 and the ambient atmosphere, and the dissipating seat 50 dissipates the internal heat to the ambient atmosphere. As a result, the environment influence outside the reaction chamber 56 can be efficiently precluded, and a stable temperature gradient can be formed in the reaction chamber 56, such that the insulated isothermal polymerase chain reaction can be performed stably.

In order to establish a specific temperature gradient in the test tube 12 helpful for performing PCR, the through hole 54 of the dissipating seat 50 is provided with a relatively big diameter section 541 and a relatively small diameter section 542 located below the relatively big diameter section 541. In this way, the heat dissipation of the PCR mixed solution at the region corresponding to the relatively small diameter section 542 will be higher than that at the region corresponding to the relatively big diameter section 541. It is revealed by experiments that the configuration of the dissipating seat 50 provided by the present invention makes PCR more efficient. The aforesaid experiments for PCR were conducted in seven different environmental temperatures ranging from 10° C. to 40° C. with a condition that the temperature of the heating seat 30 is set at a range of 104° C. to 115° C. for heating the PCR mixed solution inside the bottom 121 of the test tube 12 to a temperature of 93° C. to 97° C. The temperature of the dissipating seat 50 measured is in a range from 36° C. to 53° C., and the temperature at the PCR mixed solution level measured ranges from 36° C. to 53° C.; therefore, the PCR can be performed efficiently.

In order to stably mount the test tube 12 in the dissipating seat 50 and the adiabatic seat 20, a tube rack 60 can be further provided on the dissipating seat 50. The tube rack 60 is provided with a receiving hole 62 for insertion of the test tube 12. The receiving hole 62 has a shape complementary to the shape of the upper section 123 of the test tube 12, such that the test tube 12 can be stationarily set in the receiving hole 62 of the tube rack 60.

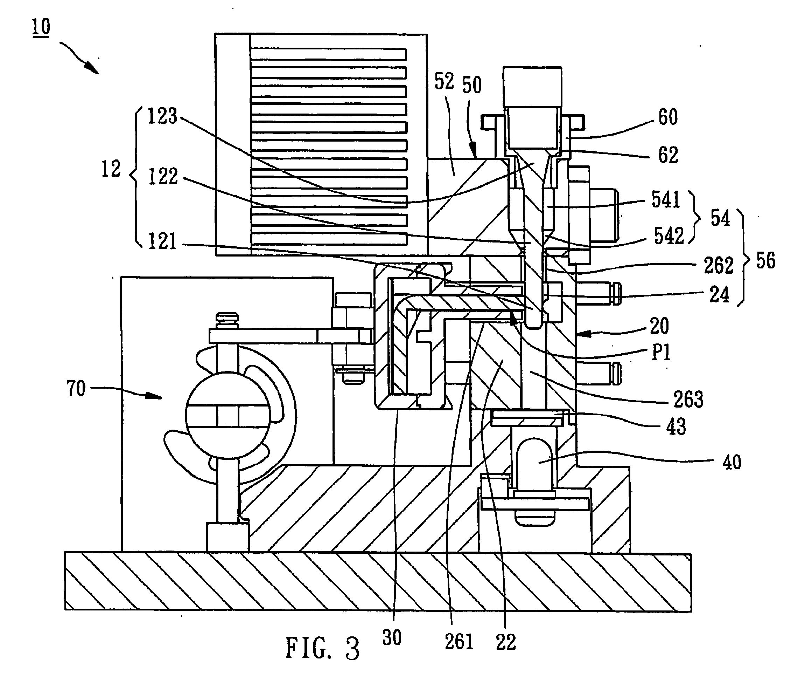

Referring to FIGS. 3 and 4, a drive 70 can be further provided to be connected with the heating seat 30 for driving the heating seat 30 to move between a contact position P1 and a release position P2. For the drive 70, a motor, pneumatic cylinder or oil cylinder can be used. When the heating seat 30 is driven by the drive 70 to move to the contact position P1, the heating seat 30 contacts the bottom 121 of the test tube 12, such that the PCR mixed solution in the bottom 121 of the test tube 12 can be heated. When the heating seat 30 is forced by the drive 70 to the release position P2, the heating seat 30 moves away from the test tube 12 to stop heating the bottom 121 of the test tube 12.

The invention being thus described, it will be obvious that the same may be varied in many ways. For example, the LED module and the filter 43 can be installed in the lower channel 263 of the adiabatic seat 20 such that the thermal convection polymerase chain reaction device 10 of the present invention can be compactly made. Further, a laser module can be used as the light unit 40, such that the filter 43 can be eliminated. Such variations are not to be regarded as a departure from the spirit and scope of the invention, and all such modifications as would be obvious to one skilled in the art are intended to be included within the scope of the following claims.

## Claims

1. A thermal convection polymerase chain reaction device (10) for holding a test tube (12) and the test tube (12) having a bottom (121), a middle section (122) and an upper section (123) in which insulated isothermal polymerase chain reaction is performed, the thermal convection polymerase chain reaction device (10) comprising: an adiabatic seat (20) having a body (22) provided with a chamber (24) for receiving the bottom (121) of the test tube (12), a side channel (261) communicated between the chamber (24) and an ambient environment, and an upper channel (262) communicated between the chamber (24) and the ambient environment for insertion of the test tube (12); and a heating seat (30) inserted into the side channel (261) for stopping at and introducing heat energy into the bottom (121) of the test tube (12), wherein the heating seat (30) is an electrical heater which contacts and heats the bottom (121) of the test tube (12), and a gap between the upper channel (262) and the test tube (12) is small so that air in said gap being heated by the heating seat (30) does not hinder heat dissipation of the middle section (122) and the upper section (123) of the test tube (12).

2. The thermal convection polymerase chain reaction device (10) of claim 1, wherein the adiabatic seat (20) is made from a plastic material or a ceramic material.

3. The thermal convection polymerase chain reaction device (10) of claim 1, wherein the heating seat (30) is made of metal.

4. The thermal convection polymerase chain reaction device (10) of claim 1, further comprising a light unit (40) located below the adiabatic seat (20); wherein the adiabatic seat (20) is provided with a lower channel (263) through which the light emitted from the light unit (40) enters into the chamber (24).

5. The thermal convection polymerase chain reaction device (10) of claim 4, further comprising a filter (43) arranged between the chamber (24) and the light unit (40); wherein the light unit (40) is an LED module, a halogen lamp, a tritium lighting unit or a xenon arc lamp.

6. The thermal convection polymerase chain reaction device (10) of claim 4, wherein the light unit (40) is a laser module.

7. The thermal convection polymerase chain reaction device (10) of claim 1, further comprising a dissipating seat (50) having a main body (52) mounted on the adiabatic seat (20) and provided with a through hole (54) in communication with the upper channel (262) of the adiabatic seat (20) for insertion of the test tube (12), the upper and middle portion of the test tube (12) received within the dissipating seat (50), the dissipating seat (50) being adapted to transmit the heat energy at the middle and upper section (123)s of the test tube (12) to the ambient atmosphere while the heating seat (30) is introducing the heat energy into the bottom (121) of the test tube (12)

8. The thermal convection polymerase chain reaction device (10) of claim 7, wherein the dissipating seat (50) is made of metal.

9. The thermal convection polymerase chain reaction device (10) of claim 7, wherein the chamber (24) of the adiabatic seat (20), the upper channel (262) of the adiabatic seat (20) and the through hole (54) of the dissipating seat (50) combinedly form a reaction chamber (56) and a heat energy in the reaction chamber (56) is transferred to the ambient environment through the dissipating seat (50).

10. The thermal convection polymerase chain reaction device (10) of claim 7, wherein the through hole (54) of the dissipating seat (50) has a relatively big diameter section (541) and a relatively small diameter section (542) located below the relatively big diameter section (541).

11. The thermal convection polymerase chain reaction device (10) of claim 7, further comprising a tube rack (60) mounted on the dissipating seat (50) and provided with a receiving hole (62) for insertion of the test tube (12).

12. The thermal convection polymerase chain reaction device (10) of any of the claims 1-11, further comprising a drive (70) connected with the heating seat (30) for moving the heating seat (30) between a contact position (P1) where the heating seat (30) contacts the bottom (121) of the test tube (12) and a release position (P2) where the heating seat (30) discontacts from the test tube (12).

## Patentansprüche

1. Eine thermische Konvektions-Polymerasekettenreaktionsvorrichtung (10) zum Aufnehmen eines Teströhrchens (12) und ein Teströhrchen (12) mit einem Boden (121), einem mittleren Abschnitt (122) und einem oberen Abschnitt (123), in dem die isolierte isotherme Polymerase-Kettenreaktion durchgeführt wird, die thermische Konvektions-Polymerasekettenreaktionsvorrichtung (10) umfasst:
einen adiabatischen Sitz (20) mit einem Körper (22), der eine Kammer (24) zur Aufnahme des Bodens (121) des Teströhrchens (12) bereitstellt, einen Seitenkanal (261) zwischen der Kammer (24) und einer Umgebung, und einen oberen Kanal (262) zwischen der Kammer (24) und der Umgebung zum Aufnehmen des Teströhrchens (12); und einen beheizbaren Sitz (30), der in den Seitenkanal (261) eingebracht ist zum Anhalten und Einführen von Wärmeenergie in den Boden (121) des Teströhrchens (12), wobei der beheizbare Sitz (30) eine elektrische Heizung ist, die den Boden (121) des Teströhrchens (12) berührt und erwärmt und ein Spalt zwischen dem oberen Kanal (262) und dem Teströhrchen (12) so klein ist, dass Luft, die in dem Spalt durch den beheizbaren Sitz (30) erhitzt wird, nicht die Wärmeabfuhr des mittleren Abschnitts (122) und des oberen Abschnitt (123) des Teströhrchens (12) behindert.

2. Die thermische Konvektions-Polymerasekettenreaktionsvorrichtung (10) nach Anspruch 1, wobei der adiabatische Sitz (20) aus einem Kunststoffmaterial oder einem Keramikmaterial hergestellt ist.

3. Die thermische Konvektions-Polymerasekettenreaktionsvorrichtung (10) nach Anspruch 1, wobei der beheizbare Sitz (30) aus Metall hergestellt ist.

4. Die thermische Konvektions-Polymerasekettenreaktionsvorrichtung (10) nach Anspruch 1, die ferner eine Lichteinheit (40) umfasst, die unterhalb des adiabatischen Sitzes (20) lokalisiert ist; wobei der adiabatische Sitz (20) einen unteren Kanal (263) bereitstellt, durch den das Licht, das von der Lichteinheit (40) emittiert wird, in die Kammer (24) tritt.

5. Die thermische Konvektions-Polymerasekettenreaktionsvorrichtung (10) nach Anspruch 4, ferner umfassend einen Filter (43), der zwischen der Kammer (24) und der Lichteinheit (40) angeordnet ist; wobei die Lichteinheit (40) ein LED-Modul, eine Halogenlampe, eine Tritium-Beleuchtungseinheit oder eine Xenonbogenlampe ist.

6. Der thermische Konvektions-Polymerasekettenreaktionsvorrichtung (10) nach Anspruch 4, wobei die Lichteinheit (40) ein Lasermodul ist.

7. Die thermische Konvektions-Polymerasekettenreaktionsvorrichtung (10) nach Anspruch 1, die ferner einen ableitenden Sitz (50) umfasst, der einen Hauptkörper (52) hat, der auf dem adiabatischen Sitz (20) gelagert ist und ein Durchgangsloch (54) besitzt, das in Kommunikation mit dem oberen Kanal (262) des adiabatischen Sitz (20) zur Aufnahme des Teströhrchens (12) steht, der obere und mittlere Teil des Teströhrchens (12) sind im ableitenden Sitz (50) aufgenommen, der ableitende Sitz (50) ist ausgebildet, um die Wärmeenergie des mittleren und oberen Abschnitts (123) des Teströhrchens (12) an die Umgebung abzuleiten, während der beheizbare Sitz (30) die Wärmeenergie in den Boden (121) des Teströhrchens (12) einführt.

8. Die thermische Konvektions-Polymerasekettenreaktionsvorrichtung (10) nach Anspruch 7, wobei der ableitende Sitz (50) aus Metall hergestellt ist.

9. Die thermische Konvektions-Polymerasekettenreaktionsvorrichtung (10) nach Anspruch 7, wobei die Kammer (24) des adiabatischen Sitz (20), der obere Kanal (262) des adiabatischen Sitz (20) und das Durchgangsloch (54) des ableitenden Sitz (50) in Kombination eine Reaktionskammer (56) bilden und eine Wärmeenergie in der Reaktionskammer (56) durch den ableitenden Sitz (50) an die Umgebung überführt wird.

10. Die thermische Konvektions-Polymerasekettenreaktionsvorrichtung (10) nach Anspruch 7, wobei das Durchgangsloch (54) des ableitenden Sitz (50) einen Abschnitt mit einem relativ großen Durchmesser (541) und einen Abschnitt mit einem relativ kleinen Durchmesser (542) aufweist, der sich unterhalb des Abschnitts mit relativ großem Durchmesser (541) befindet.

11. Die thermische Konvektions-Polymerasekettenreaktionsvorrichtung (10) nach Anspruch 7, ferner umfassend einen Röhrchenständer (60), der auf dem ableitenden Sitz (50) gelagert ist und ein Aufnahmeloch (62) zum Einsetzen des Teströhrchens (12) bereitstellt.

12. Die thermische Konvektions-Polymerasekettenreaktionsvorrichtung (10) nach einem der Ansprüche 1-11, die ferner einen Antrieb (70) umfasst, der mit dem beheizbaren Sitz (30) verbunden ist, um den beheizbaren Sitz (30) zwischen einer Kontaktposition (P1), in der der beheizbare Sitz (30) den Boden (121) des Teströhrchens (12) kontaktiert, und einer Freigabeposition (P2), in der der beheizbare Sitz (30) nicht das Teströhrchen (12) kontaktiert, zu bewegen.

## Revendications

1. Dispositif de réaction en chaîne de la polymérase par convection thermique (10) destiné à maintenir un tube à essai (12), le tube à essai (12) ayant une section inférieure (121), une section intermédiaire (122) et une section supérieure (123) dans lesquelles une réaction en chaîne de la polymérase isothermique isolée est effectuée, le dispositif de réaction en chaîne de la polymérase par convection thermique (10) comprenant : un siège adiabatique (20) qui possède un corps (22) muni d'une chambre (24) destinée à recevoir la partie inférieure (121) du tube à essai (12), un canal latéral (261) qui communique entre la chambre (24) et un environnement ambiant, et un canal supérieur (262) qui communique entre la chambre (24) et l'environnement ambiant pour l'insertion du tube à essai (12) ; et un siège chauffant (30) inséré dans le canal latéral (261) afin de s'arrêter au niveau de et d'introduire une énergie thermique dans la section inférieure (121) du tube à essai (12), le siège chauffant (30) étant un réchauffeur électrique qui touche et chauffe la section inférieure (121) du tube à essai (12), et un espace entre le canal supérieur (262) et le tube à essai (12) étant réduit de sorte que l'air présent dans ledit espace chauffé par le siège chauffant (30) ne perturbe pas la dissipation thermique de la section intermédiaire (122) et la section supérieure (123) du tube à essai (12).

2. Dispositif de réaction en chaîne de la polymérase par convection thermique (10) selon la revendication 1, dans lequel le siège adiabatique (20) est composé d'un matériau plastique ou d'un matériau céramique.

3. Dispositif de réaction en chaîne de la polymérase par convection thermique (10) selon la revendication 1, dans lequel le siège chauffant (30) est composé de métal.

4. Dispositif de réaction en chaîne de la polymérase par convection thermique (10) selon la revendication 1, qui comprend en outre une unité d'éclairage (40) située sous le siège adiabatique (20) ; dans lequel le siège adiabatique (20) est muni d'un canal inférieur (263) par lequel la lumière émise par l'unité d'éclairage (40) pénètre dans la chambre (24).

5. Dispositif de réaction en chaîne de la polymérase par convection thermique (10) selon la revendication 4, qui comprend en outre un filtre (43) placé entre la chambre (24) et l'unité d'éclairage (40) ; dans lequel l'unité d'éclairage (40) est un module à LED, une lampe halogène, une unité d'éclairage au tritium, ou une lampe à arc au xénon.

6. Dispositif de réaction en chaîne de la polymérase par convection thermique (10) selon la revendication 4, dans lequel l'unité d'éclairage (40) est un module de laser.

7. Dispositif de réaction en chaîne de la polymérase par convection thermique (10) selon la revendication 1, qui comprend en outre un siège dissipateur (50) qui possède un corps principal (52) monté sur le siège adiabatique (20) et muni d'un trou traversant (54) en communication avec le canal supérieur (262) du siège adiabatique (20) pour l'insertion du tube à essai (12), la partie supérieure et la partie intermédiaire du tube à essai (12) étant reçues dans le siège dissipateur (50), le siège dissipateur (50) étant adapté pour transmettre l'énergie thermique au niveau des sections intermédiaire et supérieure (123) du tube à essai (12) à l'atmosphère ambiante pendant que le siège chauffant (30) introduit l'énergie thermique dans la section inférieure (121) du tube à essai (12).

8. Dispositif de réaction en chaîne de la polymérase par convection thermique (10) selon la revendication 7, dans lequel le siège dissipateur (50) est composé de métal.

9. Dispositif de réaction en chaîne de la polymérase par convection thermique (10) selon la revendication 7, dans lequel la chambre (24) du siège adiabatique (20), le canal supérieur (262) du siège adiabatique (20) et le trou traversant (54) du siège dissipateur (50) forment ensemble une chambre de réaction (56), et une énergie thermique présente dans la chambre de réaction (56) est transférée vers l'environnement ambiant par le biais du siège dissipateur (50).

10. Dispositif de réaction en chaîne de la polymérase par convection thermique (10) selon la revendication 7, dans lequel le trou traversant (54) du siège dissipateur (50) possède une section à diamètre relativement grand (541) et une section à diamètre relativement petit (542) située sous la section à diamètre relativement grand (541).

11. Dispositif de réaction en chaîne de la polymérase par convection thermique (10) selon la revendication 7, qui comprend en outre un rack de tube (60) monté sur le siège dissipateur (50) et muni d'un trou de réception (62) pour l'insertion du tube à essai (12).

12. Dispositif de réaction en chaîne de la polymérase par convection thermique (10) selon l'une quelconque des revendications 1 à 11, qui comprend en outre un entraînement (70) relié au siège chauffant (30) afin de déplacer le siège chauffant (30) entre une position de contact (P1) dans laquelle le siège chauffant (30) touche la section inférieure (121) du tube à essai (12) et une position de libération (P2) dans laquelle le siège chauffant (30) ne touche pas le tube à essai (12).
